(19) Europäisches Patentamt
European Patent Office
Office européen des brevets

(11) EP 4 431 059 A1

(12) EUROPEAN PATENT APPLICATION
published in accordance with Art. 153(4) EPC

(43) Date of publication:
18.09.2024 Bulletin 2024/38

(51) International Patent Classification (IPC):
A61F 2/16 (2006.01)

(21) Application number: 23899113.7

(22) Date of filing: 20.11.2023

(52) Cooperative Patent Classification (CPC):
A61F 2/16

(86) International application number:
PCT/CN2023/132566

(87) International publication number:
WO 2024/120163 (13.06.2024 Gazette 2024/24)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 08.12.2022 CN 202211575612

(71) Applicant: Wuxi Vision Pro Ltd.
Wuxi, Jiangsu 214125 (CN)

(72) Inventors:
• LI, Shasha
  Wuxi, Jiangsu 214122 (CN)
• LIAO, Xiugao
  Wuxi, Jiangsu 214122 (CN)
• FENG, Zhenyu
  Wuxi, Jiangsu 214122 (CN)

(74) Representative: Petraz, Gilberto Luigi et al
GLP S.r.l.
Viale Europa Unita, 171
33100 Udine (IT)

(54) EXTENDED DEPTH-OF-FOCUS POSTERIOR CHAMBER PHAKIC INTRAOCULAR LENS AND PREPARATION METHOD THEREFOR

(57) The present invention discloses an expanded focal depth type implantable contact lens and a preparation method thereof. The expanded focal depth type implantable contact lens includes an optical body, a first supporting loop and a second supporting loop, wherein the optical body, the first supporting loop and the second supporting loop are of an integrated structure, are made of the same material and are integrally formed, and the optical body is located between the first supporting loop and the second supporting loop; and the optical body is composed of two optical surfaces, one optical surface is a plane, and the other optical surface is a free-form surface with a focal depth expanding function. In the present invention, the free-form surface is introduced; a focal depth expansion technology is applied to a lens intraocular lens; a focal depth is expanded; certain adjusting capacity is provided while myopia is corrected; the surface is smooth and does not change suddenly; and glare and halo are not introduced, such that presbyopia of a patient is corrected while myopia is corrected, thereby achieving an excellent visual effect.

FIG. 1

## Description

## Technical Field

The present invention relates to the technical field of optical elements, in particular to an expanded focal depth type implantable contact lens and a preparation method thereof.

## Background Art

An implantable contact lens is an implantable collamer lens (ICL), i.e., an intraocular lens, placed in the posterior chamber.

Due to poor eye care habits of people, long-term use of electronic devices, less outdoor activities and other factors, as well as long-term close eye use cause abnormal growth of the eye axis, thus causing refractive errors. With the development of science and technology and the increasing demand for vision in human beings, many correction methods have been developed to restore the ability of patients to regain their visions. Compared with a corneal laser surgery, traditional framed glasses, contact lenses and other correction methods, intraocular lenses are chosen by more and more young patients because they do not damage the cornea, do not need to be removed, and can be removed and replaced repeatedly.

However, with the increase of age, the ability of ciliary muscle adjustment of myopia patients has weakened, so presbyopia appears, resulting in patients suffering from both myopia and presbyopia. Existing products or methods (e.g., framed glasses, contact lenses, laser corneal surgery, and ICL implantation) for correcting refractive errors such as myopia, presbyopia and astigmatism cannot correct myopia and presbyopia at the same time.

Accordingly, how to design a lens intraocular lens that solves the problems of myopia and presbyopia at the same time and improve the visual quality of patients is a technical problem that needs to be solved by those skilled in the art.

## Summary of the Invention

The present invention provides an expanded focal depth type implantable contact lens and a preparation method thereof, which have the ability to adjust focal depth expansion, and to solve the problems of both myopia and presbyopia at the same time.

In order to achieve the above effects, the technical solution of the present invention is summarized as follows.

An expanded focal depth type implantable contact lens includes an optical body, a first supporting loop and a second supporting loop, wherein the optical body, the first supporting loop and the second supporting loop are of an integrated structure, are made of the same material and are integrally formed, and the optical body is located between the first supporting loop and the second supporting loop; and said optical body is composed of two optical surfaces that one optical surface is a plane, and the other optical surface is a free-form surface with a focal depth expanding function.

Further, one of said optical surfaces of the optical body satisfies a free-form surface design principle in which a determination method is as follows: an arbitrary spatial rectangular coordinate system is established by taking a vertex of said optical surface as an origin O and an optical axis as a Z-axis, and a coordinate axis X and a coordinate axis Y of the coordinate system are tangent to said free-form surface.

Further, a design process of said free-form surface is as follows:

the free-form surface is divided into a group of sub-surfaces according to a geometric iteration method, such that the free-form surface is discretized, the discretized free-form surface being represented by an envelope surface of the group of sub-surfaces; said free-form surface is rotationally symmetrical, and a sub-surface on one meridian is discussed separately to replace all the sub-surfaces;
it is known that a sub-surface $A_0$ on the free-form surface has an incident ray vector $\vec{I_0}(\sin\theta_0,\ \cos\theta_0)$ and a normal vector $\vec{N_0}(-\sin\gamma_0,\ \cos\gamma_0)$ respectively, wherein coordinates of a center point of the sub-surface $A_0$ are $(y_0,\ z_0)$, $\theta_0$ is an included angle between an incident ray and an optical axis, and $\gamma_0$ is an included angle between a normal direction of the sub-surface $A_0$ and the optical axis; when the incident ray is parallel light, that is, $\theta_0=0°$, the incident ray vector is $\vec{I_0}(0,\ 1)$;
the incident ray is focused to a focal point F on the optical axis after being refracted through the sub-surface Ao, coordinates of the focal point F are $(0, f_0)$, $f_0$ is a focal length of the focal point F, and an included angle $\theta_1$ between an emergent ray of the sub-surface $A_0$ and the optical axis is obtained according to a geometric relationship between a focal position of the sub-surface $A_0$ and the optical axis, $\theta_1$ being characterized by an equation:

$$\theta_1 = \arcsin\left(\frac{y_0}{f_0 - z_0}\right) \quad (1)$$

the incident ray and the optical axis satisfy the following relationship:

$$\begin{cases} \theta_1 + \gamma_0 = \beta_2 \\ \beta_1 = \gamma_0 \end{cases} \quad (2)$$

in which: $\beta_1$ is an incident angle of the incident ray passing through the intraocular lens, $\beta_2$ is an emergent angle of the emergent ray $\vec{I_1}$ passing through the intraocular lens, that is, the incident angle is an included angle between the incident ray and a normal of the sub-surface Ao, and the emergent angle is an included angle between the emergent ray $\vec{I_1}$ and the normal of the sub-surface $A_0$;

according to the Snell's law:

$$n_1\sin\beta_1 = n_2\sin\beta_2 \ (3)$$

in which: $n_1$ and $n_2$ are refractive indices of an incident medium and an emergent medium respectively, the refractive indices of the incident medium and the emergent medium are known quantities, and the incident medium represents the intraocular lens; an included angle $\gamma_0$ between the normal direction of the sub-surface $A_0$ and the optical axis is solved according to simultaneous equations (2) and (3), $\gamma_0$ being expressed as:

$$\gamma_0 = \arctan\left(\frac{\sin\theta_1}{\frac{n_1}{n_2} - \cos\theta_1}\right) \ (4)$$

a normal vector $\vec{N_0}$(-$\sin\gamma_0$, $\cos\gamma_0$) of the sub-surface $A_0$ is obtained according to $\gamma_0$; the normal vectors of all sub-surfaces are iteratively solved in sequence; and then, an envelope surface, i.e., a free-form surface, of all the sub-surfaces is fitted.

In the above scheme, the intraocular lens of the present invention has a plurality of focal points, and a long focal depth range.

An emergent ray $\vec{I_1}$ obtained after an incident ray is refracted by passing through the sub-surface $A_0$ may be obtained according to $\theta_1$, wherein $\vec{I_1}$ is characterized by an equation as $\vec{I_1} = C|\vec{I_0}|(sin\theta_1, cos\theta_1)$, in which C is a transmittance of the intraocular lens.

Further, an effective optical zone of said optical body has a diameter ranging from 4 mm to 6 mm. Further, the focal length $f_0$ of said optical body is determined by a dioptric power of the optical body, and the dioptric power ranges from 0 D to -30 D.

Further, said optical body is made of hydrophilic polyacrylate.

Further, said optical body has a refractive index of 1.437 at 35°C.

Further, said first supporting loop and said second supporting loop each have a thickness of 0.08 mm to 0.15 mm.

A preparation method of an expanded focal depth type implantable contact lens is used to design the expanded focal depth type implantable contact lens, the preparation method including the following steps:

S1: optical design: determining a dioptric power, an effective optical zone diameter and an expanded focal depth value of the optical body; modeling in zemax, taking a free-form surface before discretization as a basic spherical surface, optimizing a curvature r of the basic spherical surface that satisfies a dioptric power requirement of the optical body, and calculating a focal length range $f_{min}$ to $f_{max}$ of the optical body according to the dioptric power of the optical body,

$$f_{min} = {}^{1}\!/_{\phi}$$
$$f_{max} = {}^{1}\!/_{(\phi + \phi_{\text{expanded}})} (5)$$

in which: $f_{min}$ and $f_{max}$ are a minimum focal length and a maximum focal length of the optical body (1) respectively, and $\varphi$ and $\varphi_{\text{expanded}}$ are the dioptric power and an expanded dioptric power of the optical body (1) respectively;

setting a number i of geometric iterations to discretize the optimized basic spherical surface, wherein the number of sub-surfaces $A_0$ on one meridian is i and the intraocular lens is rotationally symmetrical; calculating positions of center points of all sub-surfaces $A_0$, $A_i$ representing any sub-surface on one meridian, ($y_i$, $z_i$) representing a position of the center point of any sub-surface $A_0$, and $f_i$ representing a focal length of any sub-surface $A_i$; dividing the focal length range of $f_1$ to $f_i$, absolute values from $f_1$ to $f_i$ increasing in sequence, wherein in order not to cause interference to an incident ray, pupil scaling is taken into account, pupil dependence is reduced, and the absolute values of the focal lengths $f_i$ of the sub-surfaces $A_i$ increase from the center to the outside;

modeling in matlab according to a geometric iteration method, and solving an included angle $\theta_i$ between an emergency ray of the sub-surface $A_i$ and the optical axis according to the focal length $f_i$ of the sub-surface $A_i$ and the position $A_i(y_i, z_i)$ of the center point of the sub-surface; solving $\gamma_0$ according to the equation (4), and obtaining a normal vector $\vec{N_i}$(-$\sin\gamma_i$, $\cos\gamma_i$) of the sub-surface $A_i$ through $\gamma_i$, $\gamma_i$ being used to represent an included angle between a normal direction of any sub-surface $A_i$ and the optical axis; solving normal vectors of all sub-surfaces $A_0$ on one meridian of the optical body (1) in sequence by using the geometric iteration method, and fitting an envelope surface, i.e., a free-form surface, of all sub-surfaces $A_0$ in combination with point coordinates of the sub-surface $A_0$;

S2: turning-milling machining: compiling a lathe program of a hydrophilic material according to the free-form surface designed by step S1; turning the optical body by using a diamond single-point cutting technology; compiling a milling machine program, and milling an appearance of an optic of the optical body and legs of the first supporting loop and the second supporting loop;

S3: polishing treatment: performing barrel polishing on the intraocular lens at a low temperature; and

S4: test validation: analyzing and testing the intraocular lens in a simulated eye system.

It may be understood that the normal vector of the sub-surface passes through the sub-surface and is perpendicular to a tangent plane of the sub-surface, the sub-surface is a miniature spherical surface, and the sub-surface may be solved by means of a geometric relationship of the normal vectors of the sub-surface. In step 1, a curvature r of the basic spherical surface that satisfies a dioptric power requirement of the optical body is optimized, wherein the optimization is completed by adjusting a parameter of the basic spherical surface by zemax.

The sub-surface $A_1$ has a corresponding focal length of $f_1$, and the sub-surface $A_i$ has a corresponding focal length of $f_i$.

Compared with the prior art, the technical solutions of the present invention have the following beneficial effects.

According to the present invention, a focal length expansion technology is applied to phakic intraocular lens, so that a focal depth adjusting capacity of the lens is increased on the basis of correcting myopia.

1. According to the present invention, the free-form surface is introduced; the focal depth expansion technology is applied to phakic intraocular lens; a focal depth is expanded; certain adjusting capacity is provided while myopia is corrected; the surface is smooth and does not change suddenly; and glare and halo cannot be introduced, such that presbyopia of a patient is corrected while myopia is corrected, thereby achieving an excellent visual effect.

2. According to the present invention, a spherical or aspheric surface of the ordinary phakic intraocular lens is replaced with the free-form surface, which effectively controls the lens thickness, and is more universal.

3. The phakic intraocular lens of the present invention is formed integrally, has a simple structure and good stability, is suitable for complex ophthalmic fluid environments, and is not easy to induce complications.

**Brief Description of the Drawings**

The accompanying drawings are for an illustrative purpose only and cannot be construed as limitations on the present invention. In order to better illustrate the embodiments, certain components in the accompanying drawings will be omitted, increased or reduced, and do not represent the dimension of an actual product. For those skilled in the art, it may be understood that certain well-known structures and their descriptions in the accompanying drawings may be omitted.

FIG. 1 is a schematic structural diagram of a front surface of a phakic intraocular lens provided by an embodiment of the present invention;

FIG. 2 is a schematic structural diagram of a side surface of the phakic intraocular lens provided by an embodiment of the present invention;

FIG. 3 is an optical path diagram of a sub-surface provided by an embodiment of the present invention; and

FIG. 4 is a schematic diagram of focal depth expansion provided by an embodiment of the present invention.

**Detailed Description of the Invention**

In order to make the objectives, technical solutions and advantages of the embodiments of the present invention more clearly, the technical solutions in the embodiments of the present invention will be described clearly and completely in conjunction with the accompanying drawings in the embodiments of the present invention. Apparently, the described embodiments are merely some embodiments, rather than all embodiments, of the present invention. Based on the embodiments of the present invention, all other embodiments derived by a person of ordinary skill in the art without creative efforts shall fall within the protection scope of the present invention.

Embodiment 1

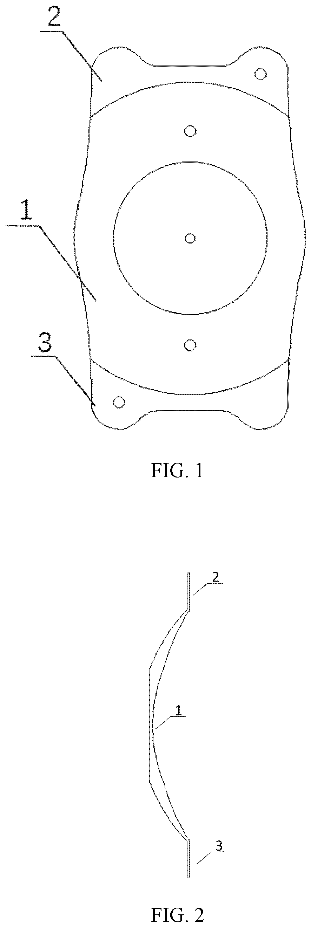

For ease of understanding, referring to FIG. 1, the present invention provides an embodiment of an expanded focal depth type implantable contact lens. The expanded focal depth type implantable contact lens includes an optical body 1, a first supporting loop 2 and a second supporting loop 3, wherein the optical body 1, the first supporting loop 2 and the second supporting loop 3 are of an integrated structure, are made of the same material and are integrally formed, and said optical body 1 is located between the first supporting loop 2 and the second supporting loop 3; and said optical body 1 is composed of two optical surfaces, one optical surface is a plane, and the other optical surface is a free-form surface with a focal depth expanding function.

The free-form surface is divided into a group of sub-surfaces according to a geometric iteration method, such that the free-form surface is discretized, the discretized free-form surface being represented by an envelope surface of the group of sub-surfaces; said free-form surface is rotationally symmetrical, and a sub-surface on one meridian is discussed separately to replace all the sub-surfaces;

it is known that a sub-surface $A_0$ on the free-form surface has an incident ray vector $\vec{I_0}(\sin\theta_0, \cos\theta_0)$ and a normal vector $\vec{N_0}(-\sin\gamma_0, \cos\gamma_0)$ respectively, wherein coordinates of a center point of the sub-surface $A_0$ are $(y_0, z_0)$, $\theta_0$ is an included angle between an incident ray and an optical axis, and $\gamma_0$ is an included angle between a normal direction of the sub-surface $A_0$ and the optical axis; when the incident ray is parallel light, that is, $\theta_0=0°$, the incident ray vector is $\vec{I_0}(0, 1)$.

the incident ray is focused to a focal point F on the optical axis after being refracted through the sub-surface Ao, coordinates of the focal point F are $(0, f_0)$, $f_0$ is a focal length of the focal point F, and an included angle $\theta_1$ between an emergent ray of the sub-surface $A_0$ and the optical axis is obtained according to a geometric relationship between a focal position of the sub-surface $A_0$ and the optical axis, $\theta_1$ being characterized by an equation:

$$\theta_1 = \arcsin\left(\frac{y_0}{f_0 - z_0}\right) \quad (1)$$

the incident ray and the optical axis satisfy the following relationship:

$$\begin{cases} \theta_1 + \gamma_0 = \beta_2 \\ \beta_1 = \gamma_0 \end{cases} \quad (2)$$

in which: $\beta_1$ is an incident angle of the incident ray passing through the intraocular lens, $\beta_2$ is an emergent angle of the emergent ray $\vec{I_1}$ passing through the intraocular lens, that is, the incident angle is an included angle between the incident ray and a normal of the sub-surface Ao, and the emergent angle is an included angle between the emergent ray $\vec{I_1}$ and the normal of the sub-surface $A_0$;

according to the Snell's law:

$$n_1\sin\beta_1 = n_2\sin\beta_2 \quad (3)$$

in which: $n_1$ and $n_2$ are refractive indices of an incident medium and an emergent medium respectively, the refractive indices of the incident medium and the emergent medium are known quantities, and the incident medium represents the intraocular lens; an included angle $\gamma_0$ between the normal direction of the sub-surface $A_0$ and the optical axis is solved according to simultaneous equations (2) and (3), $\gamma_0$ being expressed as:

$$\gamma_0 = \arctan\left(\frac{\sin\theta_1}{\frac{n_1}{n_2} - \cos\theta_1}\right) \quad (4)$$

a normal vector $\vec{N_0}(-\sin\gamma_0, \cos\gamma_0)$ of the sub-surface $A_0$ is obtained according to $\gamma_0$; the normal vectors of all sub-surfaces are iteratively solved in sequence; and then, an envelope surface, i.e., a free-form surface, of all the sub-surfaces is fitted.

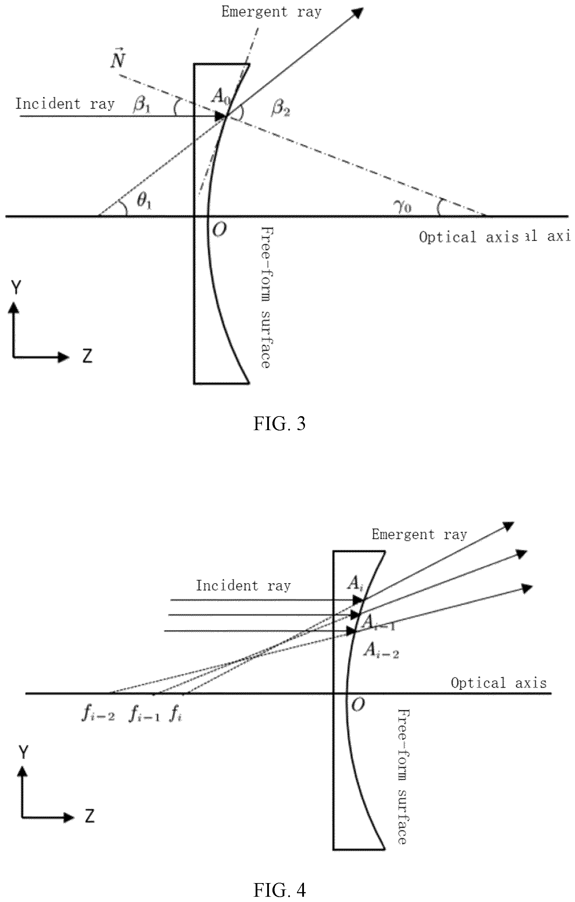

It should be noted that the included angle $\gamma_0$ between the normal direction of the sub-surface $A_i$ and the optical axis solved according to simultaneous equations (2) and (3) is solved according to equivalence relationships in the equations (2) and (3). The derivation process is as shown in FIG. 3:

$$\because \beta_1 = \gamma_0$$

$$\therefore n_1\sin\beta_1 = n_1\sin\gamma_0 = n_2\sin\beta_2$$

$$\because \theta_1 + \gamma_0 = \beta_2$$

$$\therefore n_1\sin\gamma_0 = n_2\sin(\theta_1 + \gamma_0)$$

$$\therefore \frac{n_1}{n_2}\sin\gamma_0 = \sin\theta_1\cos\gamma_0 + \sin\gamma_0\cos\theta_1$$

$$\therefore \left(\frac{n_1}{n_2} - \cos\theta_1\right)\sin\gamma_0 = \sin\theta_1\cos\gamma_0$$

$$\therefore \frac{\sin\gamma_0}{\cos\gamma_0} = \tan\gamma_0 = \frac{\sin\theta_1}{\frac{n_1}{n_2} - \cos\theta_1}$$

$$\therefore \gamma_0 = \arctan\left(\frac{\sin\theta_1}{\frac{n_1}{n_2} - \cos\theta_1}\right)$$

specifically, said optical body 1 is made of hydrophilic polyacrylate, and has a refractive index of 1.437 at 35°C.

A preparation method of an expanded focal depth type implantable contact lens is used to design the expanded focal depth type implantable contact lens, the preparation method including the following steps:

S1: optical design: determining a dioptric power, an effective optical zone diameter and an expanded focal depth value of the optical body 1; modeling in zemax, taking a free-form surface before discretization as a basic spherical surface, optimizing a curvature r of the basic spherical surface that satisfies a dioptric power requirement of the optical body 1, and calculating a focal length range $f_{min}$ to $f_{max}$ of the optical body 1 according to the dioptric power of the optical body 1,

$$f_{min} = {}^{1}/_{\phi}$$
$$f_{max} = {}^{1}/_{(\phi + \phi_{\text{expanded}})} (5)$$

in which: $f_{min}$ and $f_{max}$ are a minimum focal length and a maximum focal length of the optical body 1 respectively, and $\varphi$ and $\varphi_{\text{expanded}}$ are the dioptric power and an expanded dioptric power of the optical body 1 respectively; setting a number i of geometric iterations to discretize the optimized basic spherical surface, wherein a number of sub-surface $A_0$ on one meridian is i and the intraocular lens is rotationally symmetrical; calculating positions of center points of all sub-surfaces $A_0$, $A_i$ representing any sub-surface on one meridian, $(y_i, z_i)$ representing a position of the center point of any sub-surface $A_0$, and $f_i$ representing a focal length of any sub-surface $A_i$; dividing the focal length range of $f_1$ to $f_i$, absolute values from $f_1$ to $f_i$ increasing in sequence, wherein in order not to cause interference to an incident ray, pupil scaling is taken into account, pupil dependence is reduced, and the absolute value of the focal length $f_i$ of the sub-surface $A_i$ increases from the center to the outside; modeling in matlab according to a geometric iteration method, and solving an included angle $\theta_i$ between an emergency ray of the sub-surface $A_i$ and the optical axis according to the focal length $f_i$ of the sub-surface $A_i$ and the position $A_i(y_i, z_i)$ of the center point of the sub-surface; solving $\gamma_0$ according to the equation (4), and obtaining a normal vector $\overrightarrow{N_i}(-\sin\gamma_i, \cos\gamma_i)$ of the sub-surface $A_i$ through $\gamma_i$, $\gamma_i$ being used to represent an included angle between a normal direction of any sub-surface $A_i$ and the optical axis; solving normal vectors of all sub-surfaces $A_0$ on one meridian of the optical body 1 in sequence by using the geometric iteration method, and fitting an envelope surface, i.e., a freeform surface, of all sub-surfaces $A_0$ in combination with point coordinates of the sub-surface $A_0$;

S2: turning-milling machining: compiling a lathe program of a hydrophilic material according to the freeform surface designed by step S1; turning the optical body 1 by using a diamond single-point cutting technology; compiling a milling machine program, and milling an appearance of an optical zone of the optical body 1 and legs of the first supporting loop 2 and the second supporting loop 3;
S3: polishing treatment: performing barrel polishing on the intraocular lens at a low temperature; and
S4: test validation: analyzing and testing the intraocular lens in a simulated eye system.

According to the present invention, the freeform surface is introduced; a focal depth expansion technology is applied to the phakic intraocular lens; a focal depth is expanded; certain adjusting capacity is provided while myopia is corrected; the surface is smooth and does not change suddenly; and glare and halo are not introduced, such that presbyopia of a patient is corrected while myopia is corrected, and thus the patient can achieve a clear vision.
It should be noted that presbyopia is caused by the weakening of the patient's ability to adjust the lenses of the human eyes. The lens of the present invention provides a part of the adjusting capacity to correct the presbyopia. The adjusting capacity allows patients to still see clearly in a relatively large range of dioptric power (+1 D to +2.5 D) at an expanded focal length with fully correcting farsighted vision and without using an adjusting function of own lenses of the human eyes.

Embodiment 2

Specifically, on the basis of Embodiment 1, the scheme is explained in combination with specific embodiments, and the technical effect of this scheme is further reflected. Specifically:
a preparation method of an expanded focal depth type implantable contact lens in the present embodiment includes:

(1) design scheme: determining that a dioptric power of the intraocular lens of -10 D, an optic diameter of 5 mm, and an expanded focal depth of -1 D;
(2) optical design: modeling in zemax through the fitted dioptric power and optic area (determined by the optical zone diameter) of the intraocular lens, optimizing a curvature r=10.4 mm of a basic spherical surface, which is a concave surface, and calculating a focal length ranging from -100 mm to -111.11 mm according to the equation (5); setting a number of geometric iterations to 100, and discretizing the basic spherical surface, wherein the number of sub-surfaces on one meridian is 100 due to the rotational symmetry of the intraocular lens; calculating a center position of a group of sub-surfaces, $A_1 (y_1, z_1) \sim A_{100} (y_{100}, z_{100})$ from inside to outside respectively; and dividing the focal length range as:

$$f_1 = -100mm$$

$$f_2 = -100.11mm$$

$$f_3 = -100.22mm$$

$$...$$

$$f_{100} = -111.11mm$$

in order not to cause interference to an incident ray, pupil scaling is taken into account, pupil dependence is reduced, and the absolute values of the focal lengths $f_i$ of the sub-surfaces $A_i$ increase from the center to the outside; modeling in matlab according to a geometric iteration method, and solving an included angle $\theta_i$ between an emergency ray of the sub-surface $A_i$ and the optical axis according to the focal length $f_i$ of the sub-surface $A_i$ and the position $A_i(y_i, z_i)$ of the center point of the sub-surface; solving $\gamma_0$ according to the equation (4), and obtaining a normal vector $\overrightarrow{N_i}(-\sin\gamma_i, \cos\gamma_i)$ of the sub-surface $A_i$ through $\gamma_i$, $\gamma_i$ being used to represent an included angle between a normal direction of any sub-surface $A_i$ and the optical axis; solving normal vectors of all sub-surfaces $A_0$ on one meridian of the optical body 1 in sequence by using the geometric iteration method, and fitting an envelope surface (as shown in FIG. 4), i.e., a free-form surface, of all sub-surfaces $A_0$ in combination with point coordinates of the sub-surface $A_0$;

(3) turning-milling machining: compiling a lathe program of a hydrophilic material according to the free-form surface; turning the optical body by using a diamond single-point cutting technology; compiling a milling machine program and milling an appearance of an optic of the optical body 1 and legs of the first supporting loop 2 and the second supporting loop 3;
(4) polishing treatment: performing barrel polishing at a low temperature to obtain an intraocular lens with a qualified optical surface; and
(5) test validation: analyzing and testing the intraocular lens in a simulated eye system.

In the present embodiment, the phakic intraocular lens is introduced into an ocular model required in ISO11979-2, and an out-of-focus MTF value is obtained by means of testing with an optical device.

Embodiment 3

Specifically, on the basis of Embodiment 1, the scheme is explained in combination with specific embodiments, and the technical effect of this scheme is further reflected. Specifically:

A preparation method of an expanded focal depth type implantable contact lens in the present embodiment includes:

(1) design scheme: determining that a dioptric power of the intraocular lens of -15 D, an optic diameter of 5.5 mm, and an expanded focal depth of -1.5 D;
(2) optical design: modeling in zemax through the fitted dioptric power and optic diameter of the intraocular lens, optimizing a curvature r=6.93 mm of a basic spherical surface, which is a concave surface, and calculating a focal length ranging from -66.67 mm to -60.6 mm according to the equation (5); setting a number of geometric iterations to 150, and discretizing the basic spherical surface, wherein the number of sub-surfaces on one meridian is 150 due to the rotational symmetry of the lens; calculating a center position of a group of sub-surfaces, $A_1 (y_1, z_1)$ ~$A_{150} (y_{150}, z_{150})$ from inside to outside respectively; and dividing the focal length range as:

$$f_1 = -60.6mm$$

$$f_2 = -60.646mm$$

$$f_3 = -60.687mm$$

$$...$$

$$f_{150} = -66.667mm$$

in order not to cause interference to an incident ray, pupil scaling is taken into account, pupil dependence is reduced, and the absolute values of the focal lengths $f_i$ of the sub-surfaces $A_i$ increase from the center to the outside; modeling in matlab according to a geometric iteration method, and solving an included angle $\theta_i$ between an emergency ray of the sub-surface $A_i$ and the optical axis according to the focal length $f_i$ of the sub-surface $A_i$ and the position $A_i(y_i, z_i)$ of the center point of the sub-surface; solving $\gamma_0$ according to the equation (4), and obtaining a normal vector $\overrightarrow{N_i}(-\sin\gamma_i, \cos\gamma_i)$ of the sub-surface $A_i$ through $\gamma_i$, $\gamma_i$ being used to represent an included angle between a normal direction of any sub-surface $A_i$ and the optical axis; solving normal vectors of all sub-surfaces $A_0$ on one meridian of the optical body 1 in sequence by using the geometric iteration method, and fitting an envelope surface (as shown in FIG. 4), i.e., a free-form surface, of all sub-surfaces $A_0$ in combination with point coordinates of the sub-surface $A_0$;

(3) turning-milling machining: compiling a lathe program of a hydrophilic material according to the free-form surface; turning the optical body by using a diamond single-point cutting technology; compiling a milling machine program and milling an appearance of an optic of the optical body 1 and legs of the first supporting loop 2 and the second supporting loop 3;
(4) polishing treatment: performing barrel polishing at a low temperature to obtain an intraocular lens with a qualified optical surface; and
(5) test validation: analyzing and testing the intraocular lens in a simulated eye system.

In the present embodiment, the phakic intraocular lens is introduced into an ocular model required in ISO11979-2, and an out-of-focus MTF value is obtained by means of testing with an optical device.

Embodiment 4

Specifically, on the basis of Embodiment 1, the scheme is explained in combination with specific embodiments, and the technical effect of this scheme is further reflected. Specifically:
A preparation method of an expanded focal depth type implantable contact lens in the present embodiment includes:

(1) design scheme: determining that a dioptric power of the intraocular lens of -20 D, an optic diameter of 5 mm, and an expanded focal depth of -1.75 D;
(2) optical design: modeling in zemax through the fitted dioptric power and optic diameter of the intraocular lens, optimizing a curvature r=5.2 mm of a basic spherical surface, which is a concave surface, and calculating a focal length ranging from -50 mm to -45.98 mm according to the equation (5); setting a number of geometric iterations to 175, and discretizing the basic spherical surface, wherein the number of sub-surfaces on one meridian is 175 due to the rotational symmetry of the lens; calculating a center position of a group of sub-surfaces, $A_1 (y_1, z_1) \sim A_{175} (y_{175}, z_{175})$ from inside to outside respectively; and dividing the focal length range as:

$$f_1 = -45.977mm$$

$$f_2 = -46mm$$

$$f_3 = -46.023mm$$

$$...$$

$$f_{175} = -50mm$$

in order not to cause interference to an incident ray, pupil scaling is taken into account, pupil dependence is reduced, and the absolute values of the focal lengths $f_i$ of the sub-surfaces $A_i$ increase from the center to the outside;
modeling in matlab according to a geometric iteration method, and solving an included angle $\theta_i$ between an emergency ray of the sub-surface $A_i$ and the optical axis according to the focal length $f_i$ of the sub-surface $A_i$ and the position $A_i(y_i, z_i)$ of the center point of the sub-surface; solving $\gamma_0$ according to the equation (4), and obtaining a normal vector $\vec{N_i}(-\sin\gamma_i, \cos\gamma_i)$ of the sub-surface $A_i$ through $\gamma_i$, $\gamma_i$ being used to represent an included angle between a normal direction of any sub-surface $A_i$ and the optical axis; solving normal vectors of all sub-surfaces $A_0$ on one meridian of the optical body 1 in sequence by using the geometric iteration method, and fitting an envelope surface (as shown in FIG. 4), i.e., a free-form surface, of all sub-surfaces $A_0$ in combination with point coordinates of the sub-surface $A_0$;

(3) turning-milling machining: compiling a lathe program of a hydrophilic material according to the free-form surface; turning the optical body by using a diamond single-point cutting technology; compiling a milling machine program and milling an appearance of an optic of the optical body 1 and legs of the first supporting loop 2 and the second supporting loop 3;
(4) polishing treatment: performing barrel polishing at a low temperature to obtain an intraocular lens with a qualified optical surface; and
(5) test validation: analyzing and testing the intraocular lens in a simulated eye system.

In the present embodiment, the phakic intraocular lens is introduced into an ocular model required in ISO11979-2, and an out-of-focus MTF value is obtained by means of testing with an optical device. It may be understood that the center point and the normal vector in the above embodiment are represented by two points, which refer to a coordinate representation in a coordinate system Y-Z, and at this time, X coordinates are omitted.
Obviously, the above embodiments of the present invention are only examples given to clearly illustrate the present invention, without any limitation of implementations of the present invention. For a person of ordinary skill in the art, other different forms of changes or variations can be made on the basis of the above description. There is no need and cannot be exhaustive of all implementations. Any modification, equivalent replacement, improvement and so on made within the spirit and principle of the present invention shall be encompassed by the protection scope of the present invention.

**Claims**

1. An expanded focal depth type implantable contact lens, comprising an optical body (1), a first supporting loop (2) and a second supporting loop (3), wherein the optical body (1), the first supporting loop (2) and the second supporting loop (3) are of an integrated structure, are made of the same material and are integrally formed, and the optical body (1) is located between the first supporting loop (2) and the second supporting loop (3); **characterized in that**, the optical body (1) is composed of two optical surfaces, one optical surface is a plane, and the other optical surface is a free-form surface with a focal depth expanding function.

2. The expanded focal depth type implantable contact lens according to claim 1, wherein one of the optical surfaces of the optical body (1) satisfies a free-form surface design principle in which a determination method is as follows: an arbitrary spatial rectangular coordinate system is established by taking a vertex of the optical surface as an origin O and an optical axis as a Z-axis, and a coordinate axis X and a coordinate axis Y of the coordinate system are tangent to the free-form surface.

3. The expanded focal depth type implantable contact lens according to claim 2, wherein a design process of the free-form surface is as follows:

the free-form surface is divided into a group of sub-surfaces according to a geometric iteration method, such that the free-form surface is discretized, the free-form surface discretized being represented by an envelope surface of the group of sub-surfaces; the free-form surface is rotationally symmetrical, and a sub-surface on one meridian is discussed separately to represent all the sub-surfaces;
it is known that a sub-surface $A_0$ on the free-form surface has an incident ray vector $\vec{I_0}$ ($\sin\theta_0$,$\cos\theta_0$) and a normal vector $\vec{N_0}$($-\sin\gamma_0$, $\cos\gamma_0$) respectively, wherein coordinates of a center point of the sub-surface $A_0$ are ($y_0$, $z_0$), $\theta_0$ is an included angle between an incident ray and an optical axis, and $\gamma_0$ is an included angle between a normal direction of the sub-surface $A_0$ and the optical axis; when the incident ray is parallel light, that is, $\theta_0$=0°, the incident ray vector is $\vec{I_0}$(0, 1);
the incident ray is focused to a focal point F on the optical axis after being refracted through the sub-surface Ao, coordinates of the focal point F are (0, $f_0$), $f_0$ is a focal length of the focal point F, and an included angle $\theta_1$ between an emergent ray of the sub-surface $A_0$ and the optical axis is obtained according to a geometric relationship between a focal position of the sub-surface $A_0$ and the optical axis, $\theta_1$ being **characterized by** an equation:

$$\theta_1 = \arcsin\left(\frac{y_0}{f_0 - z_0}\right) \quad (1)$$

the incident ray and the optical axis satisfy the following relationship:

$$\begin{cases} \theta_1 + \gamma_0 = \beta_2 \\ \beta_1 = \gamma_0 \end{cases} \quad (2)$$

in which: $\beta_1$ is an incident angle of the incident ray passing through the intraocular lens, $\beta_2$ is an emergent angle of the emergent ray $\vec{I_1}$ passing through the intraocular lens, that is, the incident angle is an included angle between the incident ray and a normal of the sub-surface Ao, and the emergent angle is an included angle between the emergent ray $\vec{I_1}$ and the normal of the sub-surface $A_0$;
according to the Snell's law:

$$n_1\sin\beta_1 = n_2\sin\beta_2 \quad (3)$$

in which: $n_1$ and $n_2$ are refractive indices of an incident medium and an emergent medium respectively, the refractive indices of the incident medium and the emergent medium are known quantities, and the incident medium represents the intraocular lens; an included angle $\gamma_0$ between the normal direction of the sub-surface $A_0$ and the optical axis is solved according to simultaneous equations (2) and (3), $\gamma_0$ being expressed as:

$$\gamma_0 = \arctan\left(\frac{\sin\theta_1}{\frac{n_1}{n_2} - \cos\theta_1}\right) \quad (4)$$

a normal vector $\vec{N_0}$($-\sin\gamma_0$, $\cos\gamma_0$) of the sub-surface $A_0$ is obtained according to $\gamma_0$; the normal vectors of all sub-surfaces are iteratively solved in sequence; and then, an envelope surface, i.e., the free-form surface, of all the sub-surfaces is fitted.

4. The expanded focal depth type implantable contact lens according to claim 1, wherein the focal length $f_0$ of the optical body (1) is determined by a dioptric power of the optical body (1), and the dioptric power ranges from 0 D to -30 D.

5. The expanded focal depth type implantable contact lens according to claim 1, wherein an effective optical zone of the optical body (1) has a diameter ranging from 4 mm to 6 mm.

6. The expanded focal depth type implantable contact lens according to claim 1, wherein an expanded focal depth of the optical body (1) ranges from -1 D to -2.5 D.

7. The expanded focal depth type implantable contact lens according to claim 1, wherein the optical body (1) is made of hydrophilic polyacrylate.

8. The expanded focal depth type implantable contact lens according to claim 1, wherein the optical body (1) has a refractive index of 1.437 at 35°C.

9. The expanded focal depth type implantable contact lens according to claim 1, wherein the first supporting loop (2) and the second supporting loop (3) each have a thickness of 0.08 mm to 0.15 mm.

10. A preparation method of an expanded focal depth type implantable contact lens, which is used to prepare the expanded focal depth type implantable contact lens according to any one of claims 1 to 9, the preparation method comprising the following steps:

S1: optical design: determining a dioptric power, an effective optical zone diameter and an expanded focal depth value of the optical body (1); modeling in zemax, taking a free-form surface before discretization as a basic spherical surface, optimizing a curvature r of the basic spherical surface that satisfies a dioptric power requirement of the optical body (1), and calculating a focal length range $f_{min}$ to $f_{max}$ of the optical body (1) according to the dioptric power of the optical body (1),

$$f_{min} = {}^1\!/_\phi$$
$$f_{max} = {}^1\!/_{(\phi + \phi_{expanded})} \quad (5)$$

in which: $f_{min}$ and $f_{max}$ are a minimum focal length and a maximum focal length of the optical body (1) respectively, and $\varphi$ and $\varphi_{expanded}$ are the dioptric power and an expanded dioptric power of the optical body (1) respectively; setting a number i of geometric iterations to discretize the basic spherical surface optimized, wherein a number of sub-surfaces $A_0$ on one meridian is i and the intraocular lens is rotationally symmetrical; calculating positions of center points of all sub-surfaces $A_0$, $A_i$ representing any sub-surface on one meridian, $(y_i, z_i)$ representing a position of the center point of any sub-surface $A_0$, and $f_i$ representing a focal length of any sub-surface $A_i$; dividing the focal length range of $f_1$ to $f_i$, absolute values from $f_1$ to $f_i$ increasing in sequence, wherein in order not to cause interference to an incident ray, pupil scaling is taken into account, pupil dependence is reduced, and the absolute values of the focal lengths $f_i$ of the sub-surfaces $A_i$ increase from the center to the outside; modeling in matlab according to a geometric iteration method, and solving an included angle $\theta_i$ between an emergency ray of the sub-surface $A_i$ and the optical axis according to the focal length $f_i$ of the sub-surface $A_i$ and the position $A_i(y_i, z_i)$ of the center point of the sub-surface; solving $\gamma_0$ according to the equation (4), and obtaining a normal vector $\vec{N_i}(-\sin\gamma_i, \cos\gamma_i)$ of the sub-surface $A_i$ through $\gamma_i$, $\gamma_i$ being used to represent an included angle between a normal direction of any sub-surface $A_i$ and the optical axis; solving normal vectors of all sub-surfaces $A_0$ on one meridian of the optical body (1) in sequence by using the geometric iteration method, and fitting an envelope surface, i.e., a free-form surface, of all sub-surfaces $A_0$ in combination with point coordinates of the sub-surface $A_0$;

S2: turning-milling machining: compiling a lathe program of a hydrophilic material according to the free-form surface designed by step S1; turning the optical body (1) by using a diamond single-point cutting technology; compiling a milling machine program, and milling an appearance of an optical zone of the optical body (1) and legs of the first supporting loop (2) and the second supporting loop (3);
S3: polishing treatment: performing barrel polishing on the intraocular lens at a low temperature; and
S4: test validation: analyzing and testing the intraocular lens in a simulated eye system.

FIG. 1

FIG. 2

FIG. 3

FIG. 4

## INTERNATIONAL SEARCH REPORT

| International application No. |
| --- |
| **PCT/CN2023/132566** |

**A. CLASSIFICATION OF SUBJECT MATTER**

A61F2/16(2006.01)i

According to International Patent Classification (IPC) or to both national classification and IPC

**B. FIELDS SEARCHED**

Minimum documentation searched (classification system followed by classification symbols)

IPC: A61F

Documentation searched other than minimum documentation to the extent that such documents are included in the fields searched

Electronic data base consulted during the international search (name of data base and, where practicable, search terms used)

CNTXT; ENTXTC; VEN; ENTXT; CNKI: 调节, 多焦点, 后房, 焦深, 扩展, 平面, 曲面, 拓展, 相切, posterior, chamber, crystal, multi, focus, curv+, extend+

**C. DOCUMENTS CONSIDERED TO BE RELEVANT**

| Category* | Citation of document, with indication, where appropriate, of the relevant passages | Relevant to claim No. |
| --- | --- | --- |
| PX | CN 115844587 A (WUXI VISION PRO LTD.) 28 March 2023 (2023-03-28) claims 1-10, and description, paragraphs [0006]-[0135], and figures 1-4 | 1-10 |
| X | CN 114010371 A (TIANJIN CENTURY HEALTHCARE BIOMEDICAL ENGINEERING CO., LTD.) 08 February 2022 (2022-02-08) description, paragraphs [0004]-[0070], and figures 1-8 | 1, 2, 4-9 |
| X | CN 113180888 A (WUXI VISION PRO LTD.) 30 July 2021 (2021-07-30) description, paragraphs [0008]-[0072], and figures 1-3 | 1, 2, 4-9 |
| X | US 2016193037 A1 (STAAR SURGICAL COMPANY) 07 July 2016 (2016-07-07) description, paragraphs [0070]-[0137], and figures 2-8B | 1, 2, 4-9 |
| A | US 2020375727 A1 (PHYSIOL) 03 December 2020 (2020-12-03) entire document | 1-10 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **19 February 2024** | **02 March 2024** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT
### Information on patent family members

International application No.

**PCT/CN2023/132566**

| Patent document cited in search report | | | Publication date (day/month/year) | Patent family member(s) | | | Publication date (day/month/year) |
|---|---|---|---|---|---|---|---|
| CN | 115844587 | A | 28 March 2023 | None | | | |
| CN | 114010371 | A | 08 February 2022 | None | | | |
| CN | 113180888 | A | 30 July 2021 | None | | | |
| US | 2016193037 | A1 | 07 July 2016 | US | 10485655 | B2 | 26 November 2019 |
| US | 2020375727 | A1 | 03 December 2020 | US | 11109958 | B2 | 07 September 2021 |

Form PCT/ISA/210 (patent family annex) (July 2022)